# EUROPEAN PATENT APPLICATION

(11) **EP 3 437 700 A1**
(43) Date of publication of application: **06.02.2019**
(21) Application number: 16895903.9
(22) Date of filing: 30.03.2016
(51) Int. Cl.: A63B 71/06, G06F 1/16

(54) **SMART GARMENT AND TRAINING METHOD**

(71) Applicant: Shenzhen Royole Technologies Co., Ltd., Shenzhen, Guangdong 518052 (CN)
(72) Inventor: JIANG, Chao, Shenzhen Guangdong 518052 (CN); YANG, Fan, Shenzhen Guangdong 518052 (CN); YANG, Songling, Shenzhen Guangdong 518052 (CN); XU, Rui, Shenzhen Guangdong 518052 (CN); ZHAN, Yan, Shenzhen Guangdong 518052 (CN)
(74) Representative: Hamer, Christopher K.
(86) International application number: PCT/CN2016/077869
(87) International publication number: WO 2017/166122

(57) **Abstract**

A smart garment includes a number of motion sensors (10) configured to monitor motion data of a human body, a number of prompting devices (20) and a controller (30). The motion sensors (10) and the prompting devices (20) are electrically connected to the controller (30). The controller (30) is configured to control, according to the motion data sensed by the motion sensors (10), the prompting device (20) to issue prompts. The smart garment can monitor the movements of an exerciser all the time, and then correct the movements of the exerciser, so as to achieve the objective of training.

## Description

### TECHNICAL FIELD

The present disclosure relates to the field of electronic technology, and more particularly relates to a smart garment and a training method.

### BACKGROUND

With the improvement of people's quality of life, people pay more and more attention to sports and health. Some people exercise to shape, and some people exercise to keep fit. However, improper exercise may not only fail to shape or exercise the body, but also endanger human health. At present, in order to ensure the correctness of the exercise, most people choose fitness trainers, and have to pay a lot to them. Since the movement of the exercisers cannot be monitored at all times, it is necessary to provide a smart garment that can monitor the movements of the exercisers all the time, and can provide a prompt according to the movements.

### SUMMARY

Embodiments of the present disclosure provide a smart garment and its training method, which can monitor the movements of an exerciser, and can provide a prompt according to the movements.

In a first aspect, an embodiment of the present disclosure provides a smart garment, which includes a number of motion sensors configured to monitor motion data of a human body, a number of prompting devices and a controller. The motion sensors and the prompting device are electrically connected to the controller. The controller is configured to control, according to the motion data sensed by the motion sensors, the prompting devices to issue prompts.

In a second aspect, an embodiment of the present disclosure provides a training method which is applied to the smart garment described in the first aspect. The training method includes: acquiring motion data of a human body using motion sensors of the smart garment; issuing a prompt using prompting devices, according to the motion data.

It can be seen that, in the embodiments of the present disclosure, multiple motion sensors for monitoring motion data of a human body, a controller and multiple prompting devices are added to the sportswear. When the motion sensors sense the motion data, the controller controls, according to the motion data, the prompting device to perform a prompting operation, thereby monitoring the movements of an exerciser all the time, and then correcting the movements of the exercisers, in order to achieve the objective of training.

### BRIEF DESCRIPTION OF THE DRAWINGS

To better illustrate the technical solutions of embodiments of the present disclosure or of the related art, the following descriptions will briefly illustrate the accompanying drawings described in the embodiments or in the related art. Obviously, the following described accompanying drawings are merely some embodiments of the present disclosure. Those skilled in the art can obtain other accompanying drawings according to the described accompanying drawings without creative efforts.
FIG. 1 is a structure schematic diagram of a smart garment according to a first embodiment of the present disclosure.
FIG. 2 is a structure schematic diagram of the smart garment according to a second embodiment of the present disclosure.
FIG. 3 is a flow chart of a training method according to an embodiment the present disclosure.

### DETAILED DESCRIPTION OF ILLUSTRATED EMBODIMENTS

In order to make the objectives, technical solutions, and advantages of the embodiments of the present invention more clearly, the technical solutions of embodiments of the present disclosure will be described clearly and completely in combination with the accompanying drawings of the embodiments of the present disclosure. Obviously, the described embodiments are merely a part of embodiments of the present disclosure, but not all of the embodiments. All other embodiments obtained by those skilled in the art without creative efforts based on the embodiments of the present disclosure shall fall within the protection scope of the present disclosure.

Unless otherwise defined, all technical terms or scientific terms used herein have the same meaning as commonly understood by those skill in the art to which the present disclosure belongs. Terms "first", "second", "third", "fourth" and so on used in the present disclosure are used to distinguish different objects, rather than describe specific sequences, quantity, or importance. Similarly, terms "a", "an", "the" and so on do not indicate a quantitative limitation, but merely to indicate the existence of at least one. Terms "including", "comprising" and so on mean that the element or article preceding the terms covers elements or items and their equivalents that appear after the terms, but do not exclude other elements or items. Terms "connected", "connect" or similar terms are not limited to physical or mechanical connections, but may include electrical connections, whether direct or indirect. Terms "above", "below", "left", "right", and so on are used only to indicate a relative positional relationship, and the relative positional relationship may also change correspondingly when the absolute position of the described object is changed.

The "embodiment" mentioned herein means that particular features, structures, or characteristic described with reference to the embodiments may be included in at least one embodiment of the present disclosure. Phrases appearing at various positions of the specification neither always refer to the same embodiment, nor separate or alternative embodiments that are mutually exclusive with other embodiments. It is explicitly and implicitly understood by those skilled in the art that the embodiments described herein may be combined with other embodiments.

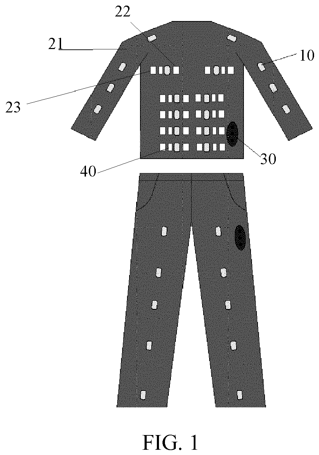

FIGs. 1 to 2 illustrate a structure schematic diagram of a smart garment which is provided in the embodiments of the present disclosure. The smart garment includes a number of motion sensors 10 configured to monitor motion data of a human body, a number of prompting devices 20, and a controller 30. The motion sensors 10 and the prompting devices 20 are electrically connected to the controller 30. The controller 30 is configured to control, according to the motion data sensed by the motion sensors 10, the prompting devices 20 to issue prompts. It can be seen that, the smart garment can monitor the movements of an exerciser all the time, and then correct the movements of the exerciser, in order to achieve the objective of training.

Alternatively, the motion sensors 10 include multiple motion sensors which are arranged in a matrix pattern. The motion sensors 10 can be arranged, according to different types and data required to be monitored, at different positions of the smart garment. For example, pulse sensors are arranged on the sleeve, heart rate sensors are arranged on the chest, and so on. Of course, the motion sensors 10 can also be directly fixed on the human body by straps or the like, to acquire more accurate monitoring results.

Alternatively, the prompting devices 20 include a flexible display screen 21, the flexible display screen 21 may be fixed on the outer surface of the smart garment by means of viscose, sewing, and so on. The flexible display screen 21 may cover the entire outer surface of the smart garment, or may also be located, according to actual needs, in certain areas of the smart garment, such as chest, back, arms, front waist, legs, knees, and so on.

It should be understood that, the smart garment of the present disclosure, includes not only tops, but also all wearable clothing such as collar, pants, hats, wristbands, leggings, shoes, and so on, as well as all deformation clothes, for example, the tops include shirts, long-sleeved shirts, short-sleeved shirts, vest, bra, and so on, the pants include short pants, trousers, underpants, and so on.

Alternatively, the prompting device 20 includes a number of speakers 22 and a number of vibrators 23 arranged on the smart garment. As illustrated in FIG. 1, the speakers 22 and the vibrators 23 are arranged in a matrix pattern.

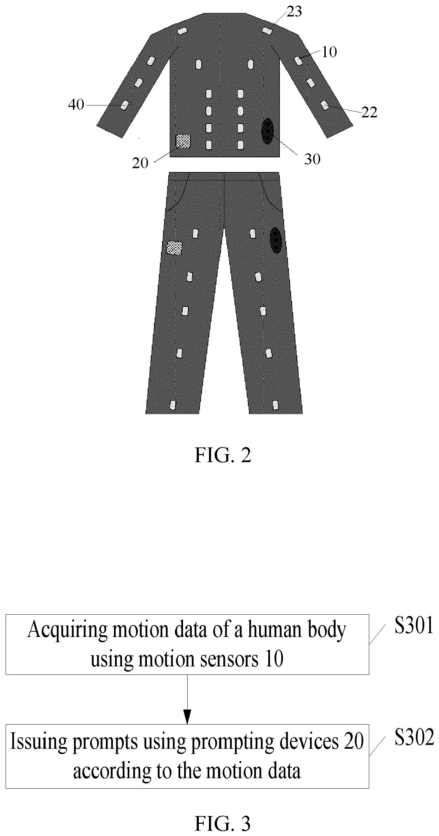

Alternatively, in order to avoid too many devices being arranged on the garment, as illustrated in FIG. 2, the motion sensors 10, the speakers 40, and the vibrators 50 may be each integrated together.

Alternatively, the controller 30 is configured to compare the motion data with a reference data range, and control the prompting device 20 to issue a prompt when the motion data is outside of the reference data range.

Alternatively, when the motion data is smaller than the reference data range, the controller 30 is configured to control the prompting device 20 to issue a first prompt; when the motion data is greater than the reference data range, the controller 30 is configured to control the prompting device 20 to issue a second prompt. The first prompt is different from the second prompt.

Alternatively, when the motion data is within the reference data range, the controller 30 is configured to control the prompting device 20 to issue a third prompt, which is different from the first prompt and the second prompt.

Alternatively, the controller 30 is configured to control the prompting device 20 to issue a prompt at the location of the corresponding motion sensor 10.

Alternatively, the motion sensors 10 include multiple muscle strength sensors. The motion data sensed by the muscle strength sensors includes exercise muscle strength. The specific implementation way of the controller 30 being configured to control the prompting device 20 to issue a prompt at the position of the corresponding motion sensor 10 may include at least one of the followings: the controller 30 is configured to control, according to the exercise muscle strength, the flexible display 21 to display a color corresponding to the exercise muscle strength at the position corresponding to the exercise muscle strength; alternatively, the controller 30 is configured to control, according to the exercise muscle strength, the speaker 22 corresponding to the exercise muscle strength to issue a voice prompt with the voice volume corresponding to the exercise muscle strength; alternatively, the controller 30 is configured to control, according to the exercise muscle strength, the vibrator 23 corresponding to the exercise muscle strength to issue a vibration prompt with vibration amplitude corresponding to the exercise muscle strength.

For example, when the exerciser is exercising, if the posture is incorrect, and when the exercise muscle strength of a certain part of the muscles is greater than the reference exercise muscle strength range, the controller 30 may control the flexible display screen 21 to issue a first prompt at the position corresponding to the certain part of the muscle, for example, display red at the position corresponding to the certain part of the muscle; alternatively, when the exercise muscle strength of the certain part of the muscles is less than the reference exercise muscle strength range, the controller 30 may control the flexible display screen 21 to issue a second prompt at the position corresponding to the certain part of the muscle, for example, display green at the position corresponding to the certain part of the muscle; alternatively, a continuous color distribution can be set according to the intensity of the exercise muscle strength, for example, the greater the exerting strength, the closer to red, on the contrary, the closer to yellow. If the posture is incorrect, the exercise muscle strength of the certain part of the muscles falls within the reference exercise muscle strength range, and the controller 30 may control the flexible display screen 21 to issue a third prompt at the position corresponding to the certain part of the muscle, for example, display white at the position corresponding to the certain part of the muscle. Of course, issuing a prompt through the flexible display screen 21 is not limited to prompting with color, but may also prompting with displaying a pattern at the position of the corresponding motion sensor 10. For example, an upward arrow is shown when the exercise muscle strength is overexert, a downward arrow is shown when the exercise muscle strength is insufficient, and a horizontal arrow is shown when the exercise muscle strength is normal. For another example, a square is shown when the exercise muscle strength is overexert, a triangle is shown when the exercise muscle strength is insufficient, and a circle is shown when the exercise muscle strength is normal.

For another example, under normal circumstances, the content displayed on the flexible display screen 21 on the back side cannot be seen by the exerciser. When the exercise muscle strength of a certain part of the muscles on the back of the body is overexert, or the exercise muscle strength of a certain part of the muscles is insufficient, in this case, the controller 30 may control the vibrator 23 corresponding to the certain part of the muscles to perform vibration prompting. When the exercise muscle strength of a certain part of the muscles on the back of the body and a certain part of the muscles in front of the body are overexert, or the exercise muscle strength of a certain part of the muscles is insufficient, in this case, the controller 30 may control the vibrator 23 corresponding to the certain part of the muscles on the back to perform vibration prompting, and controls the flexible display screen 21 to display a color corresponding to the exercise muscle strength at the position corresponding to the certain part of the muscles in front of the body. In order to distinguish between insufficient muscle strength and overexert muscle strength, the amplitude of vibration can be set. For example, a slight vibration indicates that the muscle strength is too little, and a violent vibration indicates that the muscle strength is overexert.

For another example, under normal circumstances, the content displayed on the flexible display screen 21 on the back side cannot be seen by the exerciser. When the exercise muscle strength of a certain part of the muscles on the back of the body is overexert, or the exercise muscle strength of a certain part of the muscles is insufficient, in this case, the controller 30 may control the speaker corresponding to the certain part of the muscles to perform voice prompting. When the exercise muscle strength of a certain part of the muscles on the back of the body and a certain part of the muscles in front of the body are overexert, or the exercise muscle strength of a certain part of the muscles is insufficient, in this case, the controller 30 may control the speaker corresponding to the certain part of the muscles on the back to perform voice prompting, and controls the flexible display screen 21 to display a color corresponding to the exercise muscle strength at the position corresponding to the certain part of the muscles in front of the body. To make it easy to distinguish between insufficient muscle strength and overexert muscle strength, the voice volume of the voice prompting can be set. For example, a low voice volume indicates that the muscle strength is too little, and a high voice volume indicates that the muscle strength is overexert. Of course, it is not necessary to provide a large number of speakers on the garment, but only one speaker may be provided, which can directly indicate where the muscle strength is incorrect.

Alternatively, as illustrated in FIG. 1, the motion sensors 10 of the smart garment further include multiple position sensors 40 arranged on the smart garment. The multiple position sensors 40 are arranged in a matrix pattern. The controller 30 is further configured to perform a prompting operation according to the position data sensed by the multiple position sensors 40.

Alternatively, in order to avoid too many devices being arranged on the garment, as illustrated in FIG. 2, the multiple motion sensors 10 (for example, multiple muscle strength sensors), the multiple speakers 22, the multiple vibrators 23 and the multiple position sensors 40 may be integrated together.

Alternatively, the position data includes exercise postures. When the exercise posture is incorrect, the controller 30 is configured to control at least one of the flexible display screen 21, the speakers 22, and the vibrators 23 to perform the prompting operation. The prompting operation may refer to the above-described prompting ways, and will not be described herein.

Alternatively, the smart garment can further use the flexible display screen 20 for training purpose. For example, the flexible display screen displays prompts in advance at the positions where the exerciser needs to exert strength, to prompt the exerciser to exert strength at these positions. For example, in gymnastics training, the next action needs both the wrists and elbows to exert strength at the same time, in this case, the flexible display 21 will display red at the position corresponding to both of the elbows and wrists, and the exerciser can exert strength using both the wrists and elbows when he/she sees the red prompt at the two positions. Further, the advance prompting function of the flexible display screen 21 can also set different prompt colors according to the intensity of the exerting strength, for example, the greater the exerting strength required, the redder the displayed color. In addition, the motion sensors 10 also sense muscle activity at the same time when the user performs an action. When the user exerts strength to a certain part of the muscles under the prompt of the flexible display 21, if the motion sensors 10 sense that the user's muscle strength is correct, the flexible display screen will display a correct prompt at the position of this part of the muscles, such as white light. In order to avoid user's confusion, the colors displayed corresponding to the correct prompt and the colors displayed corresponding to the prompt for the next action should be set to be different. Of course, it can be further differentiated by patterns, for example, the prompt of the next action is a circle, and the correct prompt is a triangle. In addition, it may also be combined with speakers or vibrators to prompt the correctness of the action, but the sound emitted by the speakers and the vibration of the vibrators should be different from the sound or vibration for distinguishing the intensity the exerting strength. Of course, the prompt of the next action may also be performed by speakers or vibrators without the flexible display 21.

Further, the motion sensors 10 further include various sensors capable of sensing motion states, such as heart rate sensors, pulse sensors, blood pressure sensors, speed sensors, acceleration sensors, gravity sensors, pressure sensors, altitude sensors, and so on, which can be used together with the flexible display 21, the speakers 22, the vibrators 23, the controller 30, and so on, to provide more accurate and comprehensive motion data cue effects. For example, if the speed sensors are included, the flexible display 21 displays red at the position corresponding to the arm when the arms are swung too fast; the flexible display 21 displays green at the position corresponding to the arm when the arms are swung too slow. For another example, if the height sensors are included, when making a jumping motion, the flexible display 21 displays green at the position corresponding to the shoes when the height sensors sense that the height of the jump is insufficient, and displays red at the position corresponding to the shoes when the height sensed is too high.

FIG. 3 is a flow chart of a prompt method including a specific implementation process of the smart garment described above. Although the smart garment described herein is performed based on the smart garment illustrated in FIG. 1 and FIG. 2, it should be noted that the specific operating environment of the prompting method illustrated in the embodiments of the present disclosure is not limited to the smart garment described above.

As illustrated in FIG. 3, the training method disclosed in the embodiments of the present disclosure specially includes the following operations.
S301, motion data of a human body is acquired by the motion sensors 10.
S302, a prompt is issued by the prompting device 20 according to the motion data.

Alternatively, the specific implementation way of a prompt being issued by the prompting device 20 according to the motion data includes: comparing the motion data with a reference data range, and issuing a prompt using the prompting device 20 when the motion data is outside of the reference data range.

Alternatively, the specific implementation way of issuing a prompt using the prompting device 20 when the motion data is outside of the reference data range includes: the prompting device 20 issuing a first prompt when the motion data is smaller than the reference data range, or the prompting device 20 issuing a second prompt when the motion data is greater than the reference data range. The first prompt is different from the second prompt.

Alternatively, the prompting device 20 issues a third prompt when the motion data is within the reference data range. The third prompt is different from the first prompt and the second prompt.

Alternatively, the specific implementation way of issuing a prompt using the prompting device 20 includes: issuing a prompt at the location of the corresponding motion sensor 10 using the prompting device 20.

Alternatively, the prompting device 20 includes a flexible display screen 21 on the surface of the wear. The operation of issuing a prompt at the location of the corresponding motion sensor 10 using the prompting device 20 includes: the flexible display screen 21 displaying a prompt at the location of the corresponding motion sensor 10.

Alternatively, the operation that the flexible display screen 21 displays a prompt at the location of the corresponding motion sensor 10 includes: when the motion data is smaller than the reference data range, the flexible display screen 21 displaying a first color at the location of the corresponding motion sensor 10, or when the motion data is greater than the reference data range, the flexible display screen 21 displaying a second color at the location of the corresponding motion sensor 10. The first color is different from the second color.

Alternatively, the prompting device 20 includes a vibrator 22 on the surface of the wear. The operation of issuing a prompt at the location of the corresponding motion sensor 10 using the prompting device 20 includes: the vibrator 22 issuing a vibration prompt at the location of the corresponding motion sensor 10.

Alternatively, the specific implementation way of the vibrator 22 issuing a vibration prompt at the location of the corresponding motion sensor 10 includes: when the motion data is smaller than the reference data range, the vibrator 22 issuing a first vibration at the location of the corresponding motion sensor 10, or when the motion data is greater than the reference data range, the vibrator 22 issuing a second vibration at the location of the corresponding motion sensor 10. The amplitude of the first vibration is lower than that of the second vibration.

For example, it is assumed that the motion sensors are integrated with a speaker, and the motion data includes exercise muscle strength. The smart garment determines, according to the exercise muscle strength, a target motion sensor that acquires the exercise muscle strength data, and determines, according to a preset mapping relationship between muscle strengths and colors, a target color corresponding to the exercise muscle strength data, and simultaneously determines the position of the target motion sensor, and then the smart garment determines, according to the position of the target motion sensor, the position to be displayed by the flexible display screen corresponding to the target motion sensor, and finally the smart garment controls the flexible display screen to display the target color at the position to be displayed.

For another example, it is assumed that the motion sensors are integrated with speakers, and the motion data includes exercise muscle strength. The smart garment determines, according to the exercise muscle strength, a target motion sensor that acquires the exercise muscle strength, and determines, according to a preset mapping relationship between muscle strengths and voice volumes, a target voice volume corresponding to the exercise muscle strength, and simultaneously determines the position of the target motion sensor, and then the smart garment determines, according to the position of the target motion sensor, a target speaker which is integrated with the target motion sensor, and finally the smart garment controls the target speaker to perform voice prompt according to the target voice volume.

For another example, it is assumed that the motion sensors are integrated with vibrators, and the motion data includes exercise muscle strength. The smart garment determines, according to the exercise muscle strength, a target motion sensor that acquires the exercise muscle strength, and determines, according to a preset mapping relationship between muscle strengths and vibration amplitudes, a target vibration amplitude corresponding to the exercise muscle strength, and simultaneously determines the position of the target motion sensor, and then the smart garment determines, according to the position of the target motion sensor, a target vibrator which is integrated with the target motion sensor, and finally the smart garment controls the target vibrator to issue a vibration prompt according to the target vibration amplitude.

It should be noted that the specific implementation way of each operations of the training method provided in the embodiments of the present disclosure may be combined with specific implementation way of the smart garment described above, and will not be described herein.

Alternatively, the method as illustrated in FIG. 3 further includes the following operations: obtaining position data which is acquired by the position sensors 40, and performing a prompting operation according to the position data.

Alternatively, the position data includes exercise postures. The specific implementation way of the smart garment performing a prompting operation according to the position data includes: based on a condition that the exercise posture is incorrect, the smart garment controlling at least one of the flexible display screen, the speakers, and the multiple vibrators to perform the prompting operation.

For example, the smart garment may further include multiple position sensors 40. The multiple position sensors 40 are configured to sense whether the exercise postures of the exerciser are correct, such as whether the hands are lifted to a predetermined height, whether the feet are bent to a sufficient angle, and so on. If the exercise postures are incorrect, the prompts may be displayed in the corresponding incorrect positions. The specific implementation ways of the prompts can be referred to the above-mentioned ways and will not be described herein.

It can be seen that, in the embodiments of the present disclosure, multiple motion sensors for monitoring motion data of a human body, a controller and multiple prompting devices are added to the sportswear. When the motion sensors sense the motion data, the controller controls, according to the motion data, the prompting device to issue a prompting operation, thereby monitoring the movements of an exerciser all the time, and then correcting the movements of the exerciser, in order to achieve the objective of training.

As described above, the foregoing embodiments are merely provided for describing the technical solutions of the present disclosure, rather than limiting the present disclosure. Although the present disclosure has been described in detail with reference to the foregoing embodiments, those skilled in the art should understand that: they may still make modifications to the technical solutions described in the foregoing embodiments, or make equivalent replacements to part or all of the technical features in the technical solutions, and that such modifications or replacements do not make the essence of the corresponding technical solutions depart from the scope of the technical solutions of the embodiments of the present disclosure.

## Claims

1. A smart garment comprising a plurality of motion sensors configured to monitor motion data of a human body, a plurality of prompting devices and a controller, wherein the motion sensors and the prompting devices are electrically connected to the controller, the controller is configured to control, according to the motion data sensed by the motion sensors, the prompting devices to issue prompts.

2. The smart garment according to claim 1, wherein the controller is configured to compare the motion data with a reference data range, and control the prompting device to issue a prompt when the motion data is outside of the reference data range.

3. The smart garment according to claim 2, wherein when the motion data is smaller than the reference data range, the controller is configured to control the prompting device to issue a first prompt; when the motion data is greater than the reference data range, the controller is configured to control the prompting device to issue a second prompt, wherein the first prompt is different from the second prompt.

4. The smart garment according to claim 3, wherein when the motion data is within the reference data range, the controller is configured to control the prompting device to issue a third prompt, which is different from the first prompt and the second prompt.

5. The smart garment according to any one of claims 1 to 4, wherein the controller is configured to control the prompting device to issue a prompt at the location of the corresponding motion sensor.

6. The smart garment according to claim 5, wherein the prompting devices comprise a flexible display screen arranged on the surface of the smart garment, the controller is configured to control the flexible display to display a prompt at the position of the corresponding motion sensor.

7. The smart garment according to claim 5, wherein the prompting device comprises a plurality of vibrators arranged on the smart garment, the controller is configured to control the vibrators to issue a vibration prompt at the position of the corresponding motion sensor.

8. The smart garment according to any one of claims 1 to 4, wherein the controller is further configured to control the prompting devices to issue a pre-prompt, according to exercise posture which needs to be performed in next action.

9. The smart garment according to any one of claims 1 to 4, wherein the motion sensors comprise a plurality of muscle strength sensors or a plurality of position sensors.

10. A training method comprising:
acquiring motion data of a human body using motion sensors;
issuing prompts using prompting devices, according to the motion data.

11. The method according to claim 10, wherein the issuing prompts using prompting devices, according to the motion data, comprises:
comparing the motion data with a reference data range;
issuing a prompt using the prompting device when the motion data is outside of the reference data range.

12. The method according to claim 11, wherein the issuing a prompt using the prompting device when the motion data is outside of the reference data range, comprises:
the prompting device issuing a first prompt when the motion data is smaller than the reference data range; or
the prompting device issuing a second prompt when the motion data is greater than the reference data range;
wherein the first prompt is different from the second prompt.

13. The method according to claim 12, further comprising:
when the motion data is within the reference data range, the prompting device issuing a third prompt, which is different from the first prompt and the second prompt.

14. The method according to any one of claims 11 to 13, wherein the issuing a prompt using the prompting device, comprises:
issuing a prompt at the location of the corresponding motion sensor using the prompting device.

15. The method according to claim 14, wherein the prompting devices comprise a flexible display screen arranged on the surface of the smart garment, the issuing a prompt at the location of the corresponding motion sensor using the prompting device comprises:
the flexible display screen displaying a prompt at the location of the corresponding motion sensor.

16. The method according to claim 15, wherein the flexible display screen displaying a prompt at the location of the corresponding motion sensor comprises:
when the motion data is smaller than the reference data range, the flexible display screen displaying a first color at the location of the corresponding motion sensor; or
when the motion data is greater than the reference data range, the flexible display screen displaying a second color at the location of the corresponding motion sensor;
wherein the first color is different from the second color.

17. The method according to claim 14, wherein the prompting devices comprise a plurality of vibrators arranged on the smart garment, the issuing a prompt at the location of the corresponding motion sensor using the prompting device comprises:
the vibrator issuing a vibration prompt at the location of the corresponding motion sensor.

18. The method according to claim 17, wherein the vibrator issuing a vibration prompt at the location of the corresponding motion sensor comprises:
when the motion data is smaller than the reference data range, the vibrator issuing a first vibration at the location of the corresponding motion sensor;
when the motion data is greater than the reference data range, the vibrator issuing a second vibration at the location of the corresponding motion sensor;
wherein the amplitude of the first vibration is lower than that of the second vibration.

19. The method according to claim 10, further comprising:
the prompting devices issuing a pre-prompt at the corresponding position of exercise posture of next action, according to the exercise posture of the next action.
